# EUROPEAN PATENT SPECIFICATION

(11) **EP 2 680 893 B1**
(45) Date of publication and mention of the grant of the patent: **05.09.2018**
(21) Application number: 12712845.2
(22) Date of filing: 28.03.2012
(51) Int. Cl.: A61L 27/34, A61L 27/50, A61L 29/08, A61L 29/14, A61L 31/10, A61L 31/14

(54) **MEDICAL DEVICE HAVING A LUBRICIOUS COATING WITH A HYDROPHILIC COMPOUND IN AN INTERLOCKING NETWORK**
MEDIZINISCHE VORRICHTUNG MIT SCHMIERBESCHICHTUNG MIT HYDROPHILER VERBINDUNG IN EINEM VERBAND
DISPOSITIF MÉDICAL AYANT UN REVÊTEMENT LUBRIFIANT PRÉSENTANT UN COMPOSÉ HYDROPHILE DANS UN RÉSEAU INTERPÉNÉTRANT

(30) Priority: 07.12.2011 US 201113313986
(43) Date of publication of application: 08.01.2014
(73) Proprietor: Abbott Cardiovascular Systems, Inc., Santa Clara, CA 95054 (US)
(72) Inventor: LIN, Tung-Liang, Temecula California 92592 (US)
(74) Representative: Boult Wade Tennant LLP
(86) International application number: PCT/US2012/030927
(87) International publication number: WO 2013/085562

(56) References cited:
- US-A1- 2005 055 044
- US-A1- 2005 170 071
- US-A1- 2009 041 923
- US-A1- 2010 114 042
- US-A1- 2011 166 647

## Description

### BACKGROUND OF THE INVENTION

This invention relates to the field of lubricious hydrophilic coatings for intracorporeal medical devices such as catheters, guidewires and embolic protection filters.

The use of medical devices within a patient may be facilitated by the presence of a lubricious surface on the device. For example, intravascular devices, such as catheters and guidewires, are more easily maneuvered within a patient's vasculature when the friction between the walls of the vessel and the intravascular device is reduced. These medical devices are often utilized to implant an intracorporeal device, commonly referred to as a stent within a patient's vasculature. The implanting of the stent may release emboli into the circulatory system, which can be extremely dangerous to the patient. Debris that is carried by the bloodstream to distal vessels of the brain may cause these cerebral vessels to occlude, resulting in a stroke, and in some cases, death. Thus, when performed in a carotid artery, an embolic protection device to capture and collect released emboli may be deployed downstream to the interventional catheter. For example, embolic protection devices in the form of filters or traps can be delivered in a collapsed configuration to a location adjacent to the interventional procedure site, radially expanded to open the mouth of the filter or trap, and after the interventional procedure has been performed, the device is collapsed for removal with the captured embolic material therein. Traditional embolic protection filters may be constructed with a filtering element which includes a number of small openings designed to capture embolic debris of a certain size while allowing blood to flow there through. It is important to minimize the occlusion of these filters to prevent blockage of blood flow downstream from the area being treated.

The friction may be reduced by coating the medical device with a hydrophilic compound which becomes slippery after adsorbing an appreciable amount of water. Consequently, the hydrophilic coating provides lubricity when the coated device is exposed to aqueous solution, as when the coated device is exposed to water prior to insertion in the patient or to the patient's blood during use. Alternatively, coatings, such as fluoropolymers, and silicone, provide lubricity to the surface of an intracorporeal device without the need for exposure to aqueous solution. However, the degree of lubricity may vary greatly depending on the nature of the lubricious coating. Hydrophilic coatings provide superior lubricity compared to hydrophobic coatings, such as silicone, when tested against a biological tissue countersurface.

In addition to lowering the coefficient of friction of the coated device, an effective lubricious coating must strongly adhere to the device surface. The lubricious coating should remain adhered to the device surface during potentially extended periods of storage, as well as in response to abrasive forces encountered during use. Poor adhesive strength is undesirable because the lost coating may be left behind inside the patient during use, with a corresponding decrease in the lubricity of the device. Typically, a trade off exists between a coating's lubricity and the coating's adhesive and cohesive strength, so that attempts to increase the durability of lubricious coatings may inadvertently decrease the lubricity of the coating. Durability is particularly an issue on the surfaces of catheters and guidewires which are subjected to significant rubbing and abrasive forces as the devices are slidably advanced through the patient's tortuous vasculature. In the case of embolic filters, while it is necessary to make the filter surface more hemocompatible in use, it must be done so in a manner which does not clog the small openings of the filter to allow proper blood flow. Accordingly, any coating placed on the filter to increase lubricity must not clog the openings of the filter. Consequently, difficulty has been encountered in providing a highly lubricious coating with long lasting lubricity on a surface of medical devices such as catheters, guidewires and embolic filters.

In US 2010/0114042 there is described an article comprising a coating, which coating comprises at least two layers, of which the inner layer is a primer layer, comprising a supporting network comprising a supporting polymer, and the outer layer is a functional layer comprising a multifunctional polymerizable compound.

In US 2009/041923 there is described a medical device having a lubricious coating on at least a section of the medical device, and a method of coating a medical device. The lubricious coating is a network of a hydrophilic compound cross-linked to itself and interlocked with a network of a cross-linked polymerized multifunctional monomer or polymer. The coating can include one or more agents which provide enhanced adhesion of the coating on the device, or which provide faster hydration of the coating and/or improved lubricity. Additionally, the lubricious coating can be provided with one or more therapeutic or diagnostic agents, and in one example the agent elutes relatively quickly in a concentrated release from the lubricious coating upon hydration of the coating.

In US 2005/0170071 there is described a lubricious composition suitable for use on a medical device, the composition including at least one alkoxylated acrylate compound having at least two acrylate groups per molecule and at least one second component which provides lubricity.

In US 2005/055044 there is described an ultraviolet curable lubricious coating including at least one lubricious polymer and at least one oxygen-insensitive crosslinkable polymer. Methods of making and using the coating and articles coated therewith are also described.

Nonetheless it would be a significant advance to provide a highly durable hydrophilic coating on a surface of a medical device to render the device highly lubricious. The present invention satisfies these and other needs.

### SUMMARY OF THE INVENTION

According to a first aspect of the present invention there is provided a medical device having a lubricious coating on at least a section of the device, the medical device being in accordance with claim 1. According to a second aspect of the present invention there is provided a method of providing a lubricious coating for a medical device comprising the steps of claim 3. The invention is directed to a medical device having a lubricious coating on at least a section of the medical device, the lubricious coating comprising a network of a hydrophilic compound cross-linked to itself and interlocked with a network of a multifunctional polymerized compound. One aspect of the invention is a method of coating a medical device with the lubricious coating. Additional aspects of the invention are directed to including one or more agents in the coating which provide enhanced adhesion of the coating on the device, or which provide faster hydration of the coating and/or improved lubricity. Additionally, the lubricious coating can be provided with one or more therapeutic or diagnostic agents, and in one embodiment the agent elutes relatively quickly in a concentrated release from the lubricious coating upon hydration of the coating during use of the device.

The lubricious coating comprises the cured reaction product of a solution mixture which is applied onto a surface of the medical device and then cured on the device. The solution mixture is formed by mixing together at least the following components: a multifunctional monomer or polymer network-forming compound, a hydrophilic compound, one or more first cross-linkers for cross-linking the multifunctional monomer or polymer, and one or more second cross-linkers,different than the first cross-linkers, for cross-linking the hydrophilic compound. The first cross-linkers preferentially cross-link the multifunctional monomer or polymer relative to the hydrophilic compound, and the second cross-linkers preferentially cross-link the hydrophilic compound relative to the multifunctional monomer or polymer. In a presently preferred embodiment, the network-forming compound is an oligomer during preparation of the solution mixture. However, it may alternatively be added to the solution mixture as a monomer (prepolymerization) or as a longer chain polymer, such that it may undergo a greater or lesser degree of polymerization on the device depending on whether it is added as a monomer, oligomer, or longer chain polymer. Irrespective of whether or not the network-forming compound is added to the solution mixture in the form of a monomer or a relatively low or high molecular weight polymer, it should be understood that the multifunctional monomer or polymer of the solution mixture is in a polymerized state in the finished coating on the device.

The cross-linkers are photo cross-linkers which initiate the cross-linking reactions in response to irradiation with light (e.g., of the ultraviolet or visible wavelengths). The terminology photo cross-linkers should be understood to refer to compounds that work by various mechanisms to cause the network-forming cross-linking, including cross-linking agents that become incorporated into the network, or alternatively, photoinitiators that form radicals that result in the cross-linking reaction.

Applied to the surface of a catheter or guidewire, the lubricious coating maintains its lubricity despite the significant rubbing and abrasive force encountered during use, and in a preferred embodiment prevents or inhibits guidewire hang-up in the catheter lumen caused when agglomerations of blood and contrast increase the frictional resistance between the device surfaces and/or decrease the guidewire clearance. In the absence of the second photo cross-linker, the resulting coating would have a significant amount of the hydrophilic compound noncross-linked and only relatively weakly mechanically contained in the polymer network. Such coatings, which may be referred to as a semi-interpenetrating network (semi-IPN) coating, typically lose significant lubricity relatively quickly compared to the coating of the invention. By including a photo cross-linker specifically for the hydrophilic compound, the resulting coating of the invention preferably provides controlled cross-linking, and facilitates optimizing the curing of the coating to ultimately provide a desired amount of lubricity and durability. For example, the duration of the curing, and the amount of the second photo cross-linker relative to the amount of the hydrophilic compound are selected such that the assembled, sterilized device has a highly lubricious yet durable coating.

While not intending to be bound by theory, it is believed that the coating formulation of the invention allows for the hydrophilic compound to become chemically interlocked by cross-linking to itself (via the second photo cross-linker) to form a true interpenetrating network with the cross-linked polymer, without having the cross-linked polymer chemically (covalently) bond to the hydrophilic compound, for enhanced durability with good lubricity. Thus, it is believed that the hydrophilic compound network and the polymer network, which are chemically formed at the same time in the same mixture, are essentially permanently mechanically interlocked together. The coating is thus unlike a semi-IPN in which a noncross-linked hydrophilic compound is non-permanently mechanically intertwined/contained in a cross-linked polymer, and unlike a coating in which a matrix or underlayer polymer is used to chemically bond to the hydrophilic compound. The coating includes an adhesion promoter which improves the adhesion of the coating onto a polymeric or metal surface of the medical device. The adhesion promoter provides sufficiently strong adhesion onto the surface of the medical device, to thereby avoid the need for a reactive primer layer underneath the coating on the surface of the medical device.

A method of providing a lubricious coating for a medical device generally comprises preparing a solution mixture of a multifunctional monomer or polymer, a hydrophilic compound, one or more first initiators which preferentially cross-links the monomer or polymer relative to the hydrophilic compound, and one or more second initiators, different than the first initiator, which preferentially cross-links the hydrophilic compound relative to the monomer or polymer, and applying a coating of the solution mixture onto the surface of at least a section of the medical device. The coating of applied solution is then cured, such that the resulting lubricious coating is a network of the hydrophilic compound cross-linked to itself and interlocked with a network of the polymerized multifunctional monomer or polymer. The hydrophilic compound is a poylvinylpyrrolidone, the second photo cross-linker is a diazido compound, the multifunctional monomer or polymer is an acrylate oligomer, and the adhesion promoter is an acid functionalized acrylate. The resulting coating comprises an acrylate network of the polymerized multifunctional acrylate cross-linked to itself and to the cross-linked acid functionalized acrylate adhesion promoter, and a hydrophilic compound network of the polyvinylpyrrolidone cross-linked to itself by the diazido photo cross-linker, such that the hydrophilic compound network is interlocked with the acrylate network. The coated device can be e-beam or ethylene oxide (EtO) sterilized without significantly decreasing the lubricity or durability of the coating. A primer coating including an adhesion promoter which improves the adhesion of the lubricous coating onto a polymeric or metal surface of the medical device is initially applied to the device. The primer coating is UV curable. The primer coating is, as disclosed in claim 1, an acid functionalized monoacrylate with photinitiators. A top or outer lubricous coating is then applied to the primer coating. This lubricious coating is a hydrophilic compound such as poylvinylpyrrolidone with a cross-linker such as a diazido compound, trimethylolpropyl triacrylate and an acid functionalized monoacrylate with photoinitiators. Alternatively, the hydrophilic compound can be polyethylene oxide without diaziado compound, trimethylolpropyl triacrylate and an acid functionalized monoacrylatewith photoinitiators. The coated device can be e-beam or ethylene oxide (EtO) sterilized without significantly decreasing the lubricity or durability of the coating.

The lubricious coating of the invention provides significant and long-lasting lubricity. As a result, when applied to a catheter, guidewire or filtering device, the lubricious coating significantly reduces the frictional forces of the guidewire and the surface of a catheter shaft during advancement or retraction within a patient's body lumen for an extended period of time and helps to prevent clogging of the filter to promote blood flow there through.

### BRIEF DESCRIPTION OF THE DRAWINGS

FIGURE 1 is an elevational view, partially in section, of a balloon catheter having a lubricious coating of the invention on the catheter shaft.
FIGS. 2, 3, and 4 are transverse cross sectional views of the catheter of FIG. 1, taken along lines 2-2, 3-3, and 4-4, respectively.
FIG.4A is a transverse cross sectional view of an alternative embodiment, in which a catheter distal tip has the lubricious coating on an inner and outer surface of the distal tip, and has a less lubricious coating on the outer surface lubricious coating.
FIG. 5 illustrates a guidewire having a lubricious coating of the invention.
FIG. 6 is a transverse cross sectional view of the guidewire of FIG. 5, taken along line 6-6.
FIG. 7 is a perspective view of an embolic protection filter device having a lubricious coating of the present invention placed on the filtering element.
FIG.8 is a transverse cross sectional view of the filtering element of FIG. 7, taken along line 8-8.

### DETAILED DESCRIPTION OF THE PREFERRED EMBODIMENTS

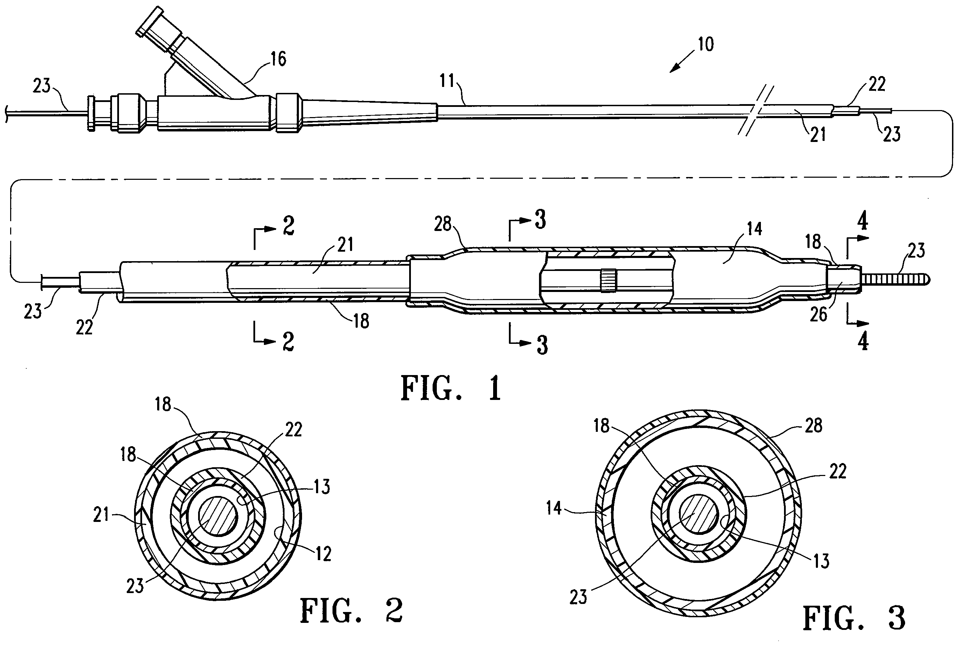

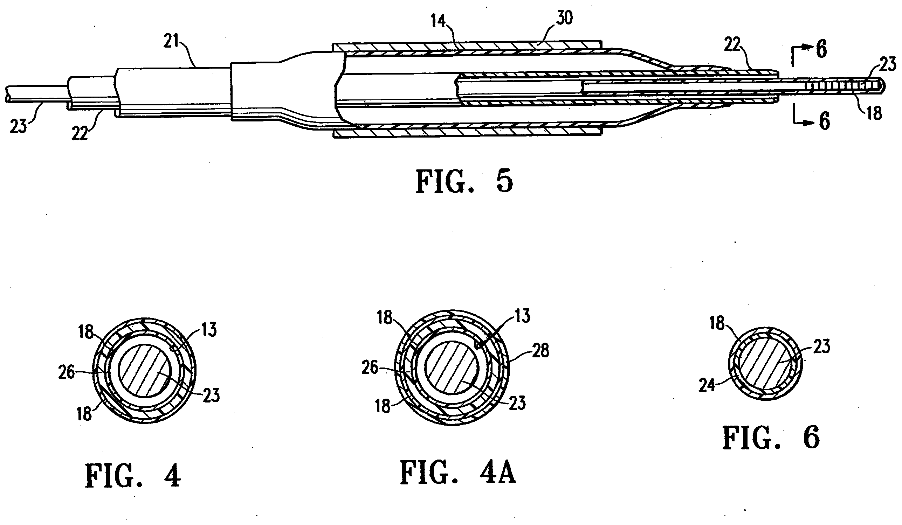

FIG. 1 illustrates one embodiment of the invention in which the medical device having a lubricious coating of the invention is a balloon catheter 10. The balloon catheter 10 generally comprises an elongated catheter shaft 11 having an inflation lumen 12 and a guidewire lumen 13 (see FIG. 2), and an inflatable balloon 14 on a distal shaft section with an interior in fluid communication with the inflation lumen. An adapter 16 mounted on the proximal end of the catheter shaft provides access to the guidewire lumen and connects to a source of inflation fluid (not shown) for inflating the balloon 14. As best shown in FIGS. 2 and 3, illustrating transverse cross sectional views of the catheter of FIG. 1 taken along lines 2-2 and 3-3, respectively, in the embodiment of FIG. 1, the shaft comprises an outer tubular member 21 having the inflation lumen 12 therein, and an inner tubular member 22 disposed in a lumen of the outer tubular member and having the guidewire lumen 13 therein configured to slidably receive a guidewire 23. The balloon 14 has a proximal skirt section sealingly secured to the distal end of the outer tubular member 21, and a distal skirt section sealingly secured to a distal end section of the inner tubular member 22, and an inflatable section there between. The catheter 10 can be advanced within a patient's body lumen, together with guidewire 23 or slidably advanced over previously introduced guidewire 23, to a desired location in the patient's body lumen, and the balloon 14 inflated to perform a medical procedure such as dilatation of a stenosis or expansion of a stent. When used as a stent delivery catheter, a stent 30 (see FIG. 5) is mounted on the balloon 14 for delivery and expansion within the patient's body lumen.

The catheter 10 has at least a section coated with a lubricious coating 18 of the invention, and more specifically has the lubricious coating 18 on at least a section of the shaft 11. In the embodiment of FIG. 1, the lubricious coating 18 is on the outer surface of the outer tubular member 21 (the outer lubricious coating), and on the inner surface of the inner tubular member 22 (see FIGS. 2 and 3), and on a distal tip section 26 of the shaft 11. The outer lubricious coating 18 can be provided on various lengths of the catheter 10, including on the entire outer length of the catheter from the proximal adapter 16 to the distal-most end of the distal tip section 26 (i.e., along the outer surface of the outer tubular member 21, the balloon 14, and the distal tip section 26), or on a shorter length, such that the outer lubricious coating 18 typically extends from the distal-most end of the catheter, proximally for at least about 25 to about 40 cm. For example, in one embodiment, the lubricious coating 18 extends along a 25 to 40 cm portion of the catheter along the outer surface of the distal tip section 26, the balloon 14, and only a distal portion of the outer tubular member 21. If the catheter 10 is used for delivery of a stent, a section of the balloon may be masked during coating, so that the stent can be mounted on a noncoated section of the balloon for good stent retention. The lubricious coating 18 on the inner surface of the inner tubular member may extend along the entire length of the inner tubular member 22 from the proximal to the distal end thereof, or along a shorter length. In embodiments in which the lubricious coating 18 is on the inner surface of the inner tubular member and the coating 18 is photo-cured, the inner tubular member is preferably formed of a polymer transparent to the radiation used to cross-link the coating 18. In the embodiment of FIG. 1, the outer surface of the balloon 14 has a coating 28, typically a lubricious coating, different than the lubricious coating 18 on the shaft 11, as discussed in more detail below. However, as discussed above, the balloon 14 can additionally or alternatively be coated with the lubricious coating 18.

The distal tip section 26 of the shaft 11 , formed by the distal end of the inner tubular member 22 and/or by a soft distal tip member secured to the distal end of the inner tubular member 22 and/or balloon proximal skirt, has the lubricious coating 18 on the outer and the inner surface thereof, as best shown in FIG. 4, illustrating a transverse cross section of the distal tip section 26 of the catheter 10 of FIG. 1, taken along line 4-4. However, in alternative embodiments, the lubricious coating 18 is located on just the outer or just the inner surface of the distal tip section 26. FIG. 4A illustrates an alternative embodiment in which the lubricious coating 18 on the outer surface of the distal tip section 26 is further coated with a second, different lubricious coating, which in the embodiment of FIG. 4A is the same lubricious coating 28 that is on the balloon. The lubricious coating 18 is sufficiently durable to remain on the distal tip section 26 during assembly of the catheter 10, so that in one embodiment, the lubricious coating 18 is provided on the distal tip section 26 of the catheter prior to assembly and processing of the catheter 10, for example by dip coating or wiping on a distal tip member before it is attached to the inner member and/or balloon. After assembly of the catheter, the second lubricious coating 28 is applied on the balloon 14 and tip 26. The undercoat of lubricious coating 18 of the invention on the distal tip 26 is provided to minimize variations, and enhance the durability of the lubricity of the distal tip 26 of the fully assembled catheter, which improves the ability of the catheter to cross tight stenosis in the patient's body lumen. In a presently preferred embodiment, the hydrophilic coating applied to the distal tip before it is attached to the catheter is the interlocking network lubricious coating 18 discussed in more detail below, although in alternative embodiments a variety of suitable hydrophilic lubricious coatings including PEO or PVP based coatings can be applied to the distal tip before it is attached to the catheter in accordance with a method of the invention.

Although illustrated in the embodiment of FIG. 1 on the outer tubular member 21, inner tubular member 22, and distal tip section 26 of the catheter 10, it should be understood that the coating 18 can alternatively be applied to fewer areas of the catheter 10 such as just the outer tubular member 21, or to different areas of the catheter 10. Thus, the lubricious coating 18 of the invention can be applied to a variety of suitable locations on the catheter 10. Additionally, the lubricious coating 18 can be applied to a variety of suitable alternative medical devices. For example, FIG. 5 illustrates the lubricious coating 18 on guidewire 23. Guidewire 23 comprises a metallic core and coiled wire distal tip, and the coating 18 is preferably along at least a distal section of the guidewire including the floppy distal tip. Guidewire 23 having the lubricious coating 18 of the invention thereon preferably advances and retracts with very low friction force within the guidewire lumen of a catheter.

As best shown in FIG. 6, illustrating a transverse cross section of the guidewire of FIG. 5, in the embodiment of FIG. 5 the guidewire has a polymer layer 24 on an outer surface of the metallic core such that the lubricious coating 18 is on an outer surface of the guidewire polymer layer 24. In one embodiment, the polymer layer 24 is a polyurethane coating or layer on a stainless steel or NiTi core wire of the guidewire, although the polymer layer 24 can be formed of a variety of polymers including polyolefin, copolyamides, copolyesters or filled polyurethane. Fillers such as tungsten, barium, and bismuth and their compounds in general can be added to enhance radiopacity.

The lubricious coating 18, on catheter 10 and/or guidewire 23, comprises the cured reaction product of a solution mixture comprising a multifunctional monomer or polymer network-forming compound; a hydrophilic compound; one or more first cross-linkers for cross-linking the multifunctional monomer or polymer, which preferentially cross-links the multifunctional monomer or polymer relative to the hydrophilic compound; and one or more second cross-linkers, different than the first cross-linkers, for cross-linking the hydrophilic compound, which preferentially cross-links the hydrophilic compound relative to the multifunctional monomer or polymer. The resulting cured coating on the medical device is a network of the hydrophilic compound cross-linked to itself and interlocked with a network of the cross-linked polymerized multifunctional monomer or polymer.

The multifunctional network-forming compound is a high molecular weight ethoxylated trimethylol propane triacrylate (ETMPTA) (e.g., PHOTOMER® 4158, available from Cognis). The ETMPTA oligomer polymerizes and cross-links during curing to form a network of cross-linked ETMPTA. Cross-linkers are photosensitive molecules (photo cross-linkers). As disclosed in claim 1, in the embodiment in which the multifunctional oligomer is a triacrylate, the solution mixture includes mixed first photoinitiators including benozophenone, and a benzil dimethyl ketal such as 2,2-dimethoxy-2-phenyl acetophenone(PHOTOMER® 51, available from Cognis) for photocuring the triacrylate. A variety of mixed first photoinitiators are typically provided, which work by different mechanisms to initiate polymerization and cross-linking of the triacrylate (and acrylates in general) as is generally known. For example, upon irradiation, PHOTOMER®51 undergoes a unimolecular bond cleavage to yield free radicals, while the benezophenone undergoes a bimolecular reaction in the presence of alcohol in which hydrogen abstraction creates hydroxyl (or ketal-type) radicals. Ultraviolet, as opposed to visible light, photoinitiation is preferred for faster curing time.

A presently preferred hydrophilic compound is a polyvinylpyrrolidone (PVP, (poly (N-vinyl-2-pyrrolidone)), which, when in combination with the second photo cross-linker such as a diazidostilbene (DAS) or derivative thereof, cross-links during curing to form a network of cross-linked PVP. Presently preferred PVPs include PVP K-90 and PVP K-120, available for example from ISP Chemicals, Inc., the K number being significant as it is related to the molecular weight of the PVP. Preferred cross-linkable PVPs have a relatively high molecular weight of greater than about 1 ,000,000 g/mole for cross-linking to form the desired (lubricious) network. A presently preferred diazidostilbene for preferentially cross-linking the PVP is 4,4'-diazido-2, 2'-stilbene disulfonic acid disodium salt. Other possible diazido based second photo cross-linkers that could be used include diazidostilbene derivatives including those set forth in U.S. Patent No. 5,041,570. Upon irradiation, DAS (a photo cross-linking agent) forms a highly reactive intermediate nitrene group on both ends, and then the nitrene groups on the DAS will react with PVP to form the cross-linked network of PVP. In accordance with the invention, the DAS preferentially cross-links the PVP relative to the multifunctional monomer or polymer network-forming compound (e.g., the triacrylate). That is, the DAS cross-links PVP polymer chains together, substantially without cross-linking the polymer chains of the multifunctional polymerized monomer or polymer. Similarly, the first photo cross-linkers are not expected to cross-link the hydrophilic compound (PVP) of the coating of the invention. Additionally, curing the coating does not cross-link, graft or otherwise chemically bond the hydrophilic compound to the polymerized monomer or polymer, or to the substrate. Thus, although a variety of hydrophilic compounds are well known for use in lubricious coatings for medical devices, in the coating of the invention the hydrophilic compound has a specific initiator which can be added to the solution mixture to preferentially cross-link the hydrophilic compound to itself to a desired degree.

The amount of the second cross-linkers provided in the solution mixture relative to the amount of the hydrophilic compound, and the duration of the curing is sufficient to form a three dimensional cross-linked network of the hydrophilic compound, although the hydrophilic compound is cross-linked to a greater or less degree depending on the desired performance characteristics of the lubricious coating 18. The control provided by the invention over the cross-linking of the hydrophilic compound facilitates creating a desired lubricity and durability which can be tailored for different applications. Thus, PVP that is part of the network in lubricious coating 18 has a greater or lesser degree of cross-linking. Additionally, some noncross-linked hydrophilic compound (i.e., PVP that is not cross-linked and thus not part of the network) or a noncross-linked secondary hydrophilic compound such as PEO are present in the lubricious coating in some embodiments, for enhanced lubricity at the potential expense of durability. Specifically, network lubricious coatings in which durability and not lubricity was at issue would cross-link the hydrophilic compounds to a greater degree to maximize the durability of the coating at the expense of the lubricity, which may be acceptable in some applications. Additionally, because the cross-linking of the hydrophilic compound is more readily controllable in the lubricious coating of the invention, the amount of cross-linking caused by initially photo-curing the coating on the device can be tailored to compensate for any additional cross-linking that may occur later, as for example when sterilizing the coated device by e-beam or EtO sterilization causes further cross-linking of the coating. In one embodiment, the coated device is e-beam sterilized, and the method of coating the device involves (UV) curing the coating on the device for a relatively short duration which is insufficient to cross-link the compounds to the desired degree (e.g., as determined by performance testing of the coated medical device), and subsequently e-beam sterilizing the coated device such that the compounds further cross-link to the desired degree. Similarly, the amount of photo cross-linkers in the coating can be limited to control the amount of cross-linking caused by the photo-curing.

The solution mixture is formed by combining the multifunctional monomer or polymer, one or more hydrophilic compounds, one or more first cross-linkers, and one or more second cross-linkers together in a single solution (the compounds typically having been first dissolved in a suitable solvent before combining to form the single solution). The solution mixture is then applied to the surface of the catheter shaft 11 and/or guidewire 23, and it can be applied to the device using a variety of suitable methods including dipping, spraying, wiping the solution on the surface of the catheter or guidewire, or drawing the solution through the guidewire lumen 13 of the catheter. The coating is then typically dried on the device before the curing, and the resulting cured coating has the substantially uniform composition provided by the interlocked networks in a single layer. In one embodiment, an adhesion promoting primer is first coated onto the device and cured, and then the lubricious coating solution mixture is applied onto the cured primer. The cured coating 18 has to be hydrated to render it lubricious for use in a medical procedure. The water induction time, i.e., the time required to hydrate the coating, varies depending on the coating formulation. Thus, the terminology "lubricious coating" as used herein should be understood to refer to the finished coating on the device, either before or after the hydrophilic compound is hydrated to render the coating lubricious for use. The solution mixture includes an adhesion promoter comprising an acid functionalized acrylate which adheres to a surface of the medical device to improve adhesion of the lubricious coating 18 on the medical device. The preferred adhesion promoter bonds to the surface of the substrate (e.g., the polymer surface of the catheter shaft or the guidewire) and also cross-links to the multifunctional polymerized monomer or polymer. Thus, the first initiators preferably cross-link the adhesion promoter, such that the adhesion promoter is cross-linked to itself and to the cross-linked polymerized multifunctional monomer or polymer in the cured lubricious coating. A presently preferred adhesion promoter is PHOTOMER® 4173, an acid functionalized monoacrylate from Cognis, which bonds to a polymeric (and particularly a polyurethane) substrate layer. Alternative adhesion promoters which could be used include the acid functionalized acrylates PHOTOMER® 4703 and 4846 from Cognis. The adhesion promoter is generally 0.2% to 20%, more specifically 1% to 2%, by weight of the solution mixture. A reactive primer layer on the device, such as these acid functionalized adhesion promoters (plus a photoinitiator) are used to improve adhesion. The coating 18 of the invention adheres to the surface of the device without requiring that the hydrophilic compound is functionalized or otherwise made to reactively chemically bond to a matrix or substrate polymer.

In one embodiment, the solution mixture includes a secondary hydrophilic compound such as polyethylene oxide (PEO) which is different than the network forming hydrophilic compound (e.g., PVP). The secondary hydrophilic compound is substantially noncross-linked in the lubricious coating. Thus, an initiator which preferentially cross-links the secondary hydrophilic compound is not included in the solution mixture, and curing the coating produces relatively little or no cross-linking of the secondary hydrophilic compound. As a result of being substantially noncross-linked, the secondary hydrophilic compound preferably provides a coating which is, at least initially, more lubricious and/or which has a decreased water induction time (i.e., a quicker response to a hydration procedure). For example, a substantially noncross-linked hydrophilic compound such as polyethylene oxide (PEO) in the coating hydrates relatively quickly. Specifically, combining the first hydrophilic compound such as PVP with the secondary hydrophilic compound such as PEO or polyacrylamide provides a coating that preferably has an improved, fast water induction time after sterilization by e-beam or EtO treatment. Noncross-linked PEO or polyacrylamide preferably compensates for an increase in water induction time of the lubricious coating due to both e-beam and EtO sterilization. A variety of suitable hydrophilic compounds can be used as the secondary hydrophilic compound including PEO, polyacrylamide-co-acrylic acid and polyacrylamide. In one embodiment, a relatively small amount of the secondary hydrophilic compound is present in the coating. For example, in one embodiment, the secondary hydrophilic compound is only about 5% by weight of the amount of PVP in the lubricious coating.

In one embodiment, the solution mixture includes a dissolvable ionic compound (i.e., a salt) such as sodium chloride, and the resulting cured lubricious coating has the salt contained (dissolvably) therein at least prior to the hydration procedure used to hydrate the coating for use. The water induction time is believed to be decreased relative to the coating without the salt as a result of the presence of the salt in the cured coating.

In one embodiment, the cured lubricious coating has a therapeutic or diagnostic agent. For example, an agent added to the solution mixture is releasably contained in the cured coating such that as the cured coating swells (hydrates) during use, the agent will elute therefrom. The cured lubricious coating can be provided with a variety of agents. Anti-platelet agents, anti-thrombogenic agents, anti-coagulant agents, anti-inflammatory agents, vasodilator agents, and the like are particularly preferred for adding to the lubricious coating on the balloon 14, outer member 21, guidewire 23, and/or within the guidewire lumen 13 of the catheter shaft 11. A relatively small molecule agent such as aspirin (acetylsalicyclic acid; acetolsal) is particularly desirable in the lubricious coating because its relatively quick elution time from the lubricious coating provides a concentrated quick dose of the aspirin during the initial introduction and advancement of the catheter and/or guidewire in the patient's body lumen. Although controlled, longer term elution of agents from medical device coatings is a goal of many of prior art coatings, relatively quick, uncontrolled elution of the aspirin from the lubricious coating of the invention is desirable. The concentrated release of the aspirin from the lubricious coating upon hydration of the coating provides an anti-platelet affect during positioning of the catheter in the body lumen, which further reduces guidewire hang-up in the catheter guidewire lumen. Although aspirin has a small molecular weight (e.g., 180 g/mol), alternative agents with larger molecular weights than aspirin can alternatively be used in a coating of the invention, such as Hirudin (about 7,000 g/mol) or Heparin (about 12,000 to about 15,000 g/mol).

The lubricious coating of the invention can be provided with a variety of suitable agents (small or large molecule agents) including anti-restenosis agents, and antiinflammatory, anti-coagulating, or pro-healing drugs. The agent is typically provided by adding it into the solution mixture prior to application onto the device, which is a preferred method due to the good manufacturability, control over the amount and location of the agent on the device, and minimal disruption of the lubricity of the coating. Less preferred methods include swelling the cured coating on the device with a solution of the agent prior to use. In the embodiment illustrated in FIG. 1, the coating 28 on the balloon 14 is different than the lubricious coating 18 on the shaft. For example, the coating 28 on the balloon may be a lubricious coating which has less lubricity or may contain a different therapeutic agent than the coating on the shaft. In alternative embodiments as discussed above, the same lubricious coating 18 on the shaft 11 is provided on the balloon 14.

In one embodiment, a lubricious coating 28 on the balloon 14 has a relatively short water induction time (hydrates quickly) and includes an anti-restenosis agent such as everolimus or zotarolimus for treating artery disease and/or preventing restenosis. The agent is well preserved in the agent delivery lubricious coating 28 before balloon inflation, and since the water up-take by the agent delivery lubricious coating 28 occurs quickly, the agent is released immediately as the balloon 14 is inflated, for providing a sufficient dose of the agent at the desired site. Typically, the balloon prior to inflation is folded and thus protects some of the coating within the folds as the catheter is first hydrated and advanced within the blood vessel. In one embodiment, the agent delivery lubricious coating 28 on the balloon is the embodiment of the interlocking network lubricious coating described above having the noncross-linked secondary hydrophilic compound added thereto which provides a quick water induction (e.g., noncross-linked PEO in the interlocking network of cross-linked PVP and cross-linked triacrylate). As discussed above, the agent is preferably added to the solution mixture of the lubricious coating prior to coating of the balloon. The balloon having the agent delivery lubricious coating thereon is then folded or otherwise configured into a low profile configuration for advancement within the patient's body lumen.

In one embodiment, coating 28 on the balloon is a less lubricious coating than the lubricious coating 18 on the shaft, to prevent or inhibit the inflated balloon from slipping out of the desired treatment location in the patient's body lumen (commonly referred to as "watermelon seeding"). There are a number of alternate approaches to making the coating 28 on the balloon as a less lubricious coating than the lubricious coating 18 on the shaft. For example, a more dilute concentration solution of the same ingredients can be applied on the balloon after the same or more concentrated solution is applied over the shaft and balloon. As another example, a coating comprised of the solution incorporating one hydrophilic polymer (for example PEO) can be applied on the balloon, while a coating comprised of the solution incorporating a different hydrophilic polymer (for example PVP) can be applied on the shaft. As another example, the lubricious coating 28 can comprise the reaction product of a solution mixture of a binding multifunctional oligomer (or monomer or higher molecular weight polymer), a photo cross-linker for cross-linking the binding oligomer, and a hydrophilic compound without a photo cross-linker for preferentially cross-linking the hydrophilic compound of the less lubricious coating. The coating 28 on the balloon can thus be formed of the same component compounds as the coating 18 on the shaft but without the second photo cross-linkers, to result in a less lubricious coating. Although coating 28 is illustrated extending along the entire length of the balloon from the proximal to the distal ends of the balloon, it should be understood that in alternative embodiments, the coating 28 can extend along a shorter length of the balloon or beyond the ends of the balloon.

The following example illustrates a solution mixture for a lubricious coating 18 of the invention. In addition to the specific formulation (with the amount of each component expressed as a weight percent of the solution mixture) used in the following example, the Table also gives example solution weight percent ranges for the components which can be used in making coatings of the invention.

**TABLE A**

| Chemical | Specific Weight % (Formulation A) | General Weight % Range Formulations |
|---|---|---|
| Ethanol | 79.63 | about 60 10 about 80 |
| Isopropanol (IPA) | 5.53 | about 2 to about 10 |
| Water | 5.53 | about 2 to about 10 |
| PVP K-90 | 6.30 | about 2 to about 10 |
| PHOTOMER® 4173 | 1.02 | about 0 to about 5 |
| PHOTOMER® 4158 | 1.89 | about 1 to about 5 |
| PHOTOMER® 51 | 0.019 | about 0.01 to about 0.05 |
| Benzophenone | 0.019 | about 0.01 10 about 0.05 |
| 4,4'-diazido-2,2-stilbenedisulfonic acid disodium salt hydrate | 0.063 | about 0.01 to about 0.25 |

A solution mixture of formulation A listed in the above Table A was applied by dip coating onto a guidewire which had a metallic core wire covered by a polymer layer of a tungsten filled polyurethane polymer. In a testing procedure in which the coated guidewire is repeatedly advanced and retracted within a guidewire lumen of a catheter inner tubular member having an HDPE inner surface (the inner tubular member being filled with sterile water and kept at 37°C with a 1.25" (3.175 cm) loop), the resulting frictional force caused by the movement of the coated guidewire in the guidewire lumen remained low after multiple cycles, up to 1000 cycles and after twenty four hours. The frictional force after multiple cycles was lower when compared to a guidewire otherwise the same but coated with a lubricious coating of PEO in a cross-linked acrylate (i.e., a solution mixture of isopropanol, water, PEO, trimethylolpropyl triacrylate (TMPTA), hydroxycyclohexyl phenyl ketone and benzophenone, wherein the PEO was a POLYOX WSR N12K and was about 1.6 weight percent of the solution mixture). For example, after thirty cycles, the friction force during pulling or pushing of the guidewire coated with formulation A set forth in the above Table was about 5 grams compared to about 35 to 55 grams for the comparison guidewire.

The dimensions of catheter 10 are determined largely by the size of the balloon and guidewire to be employed, the catheter type, and the size of the artery or other body lumen through which the catheter must pass or the size of the stent being delivered. Typically, the outer tubular member 21 has an outer diameter of about 0.025 to about 0.04 inch (0.064 to 0.10 cm), usually about 0.037 inch (0.094 cm), and the wall thickness of the outer tubular member 21 can vary from about 0.002 to about 0.008 inch (0.0051 to 0.02 cm), typically about 0.003 to 0.005 inch (0.0076 to 0.013 cm). The inner tubular member 22 typically has an inner diameter of about 0.01 to about 0.018 inch (0.025 to 0.046 cm), usually about 0.016 inch (0.04 cm), and a wall thickness of about 0.004 to about 0.008 inch (0.01 to 0.02 cm). The overall length of the catheter 10 may range from about 100 to about 150 cm, and is typically about 143 cm. Preferably, balloon 14 has a length about 0.8 cm to about 6 cm, and an inflated working diameter of about 2 mm to about 10 mm. The guidewire 23 typically has length of about 190 to about 300 cm, and an outer diameter of about 0.010 to about 0.035 inch (0.0254 to 0.0889 cm).

The various catheter components may be joined using conventional bonding methods such as by fusion bonding or use of adhesives. Although the shaft 11 is illustrated as having an inner and outer tubular member, a variety of suitable shaft configurations may be used including a dual lumen extruded shaft having a side-by-side lumens extruded therein. Additionally, although the embodiment illustrated in FIG. 1 is an over-the-wire type balloon catheter having a guidewire lumen extending the full length of the catheter, it should be understood that the coating 18 of the invention can be used with a variety of suitable catheters including guiding catheters having a device lumen configured for delivering catheters or other devices, or rapid-exchange type balloon catheters having a guidewire proximal port spaced distally from the proximal end of the catheter shaft.

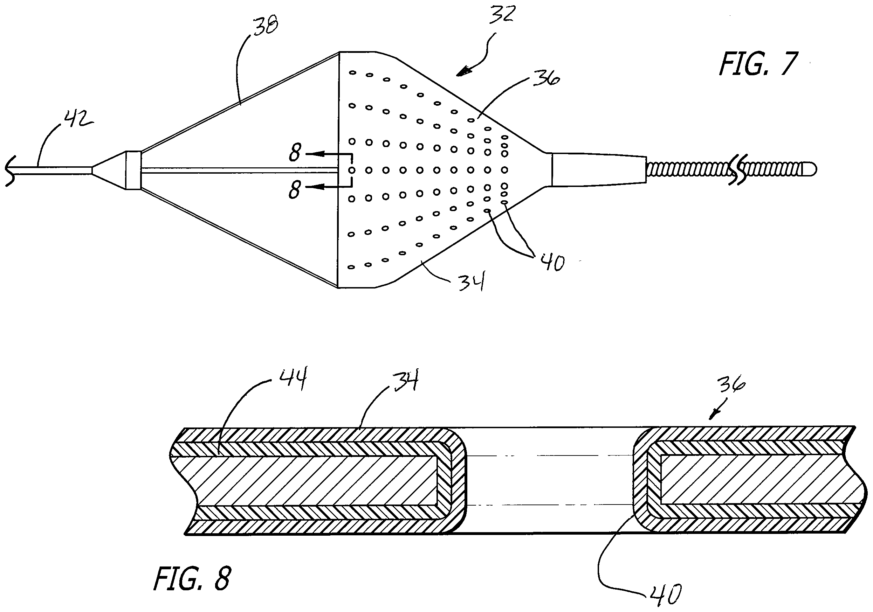

Referring now to the embodiment of FIGS. 7 and 8, a typical embolic protection device 32 is shown including a lubricious coating 34 placed on the filtering element 36 of the device. The embolic protection device 32 includes a support frame 38 to which the filtering member 36 is attached. This support frame 38 can be made from a material that is self-expanding to allow the support frame 38 to move between collapsed and expanded positions. The collapsed position can be attained, for example, by co-axially disposing a tubular sheath (not shown) over the frame 38 and filtering element 36. Retraction of the tubular sheath will allow the frame 38 to self-expand to the expanded position shown in FIG. 7. The expansion of the frame 38 causes the filtering element 36 to open, allowing the filtering element 36 to retain embolic debris that would flow into it. The filtering element 36 can be made from a polymeric material, such as Nylon 11, and includes a plurality of small outlet openings 40 formed into the material which allow blood to flow through the device to prevent blood stoppage to the area downstream from the deployed filtering device. The frame 38 and filtering element 36 can be mounted, for example, to a guidewire 42 which is used to position the filtering element in the patient's body vessel.

As can best be seen in FIG. 8, the filtering element 36 includes a lubricious coating 34 which is disposed over a primer coating 44 that is initially placed on the filtering element 36. This primer coating 44 acts as an adhesion promoter which initially bonds to the surface of the substrate (e.g., the polymeric surface of the filtering element) and also cross-links to the multifunctional polymerized monomer or polymer that forms the lubricious coating 34. This primer coating 44 is initially applied uniformly over the surface of the filtering element 36 utilizing spinning techniques and a high pressure nozzle to ensure that the openings 40 will not be occluded by the primer coating. The outer lubricious coating 34 is then applied over the primer coating 44. Again, a spinning technique may be used in applying this outer coating.

In the primer solution used to initially coat the filtering element 36, the major ingredients are an acid functionalized monoacrylate (Photomer 4173) with photoinitiators. In the top or outer hydrophilic coating solution, the major ingredients are PVP with diazido compound or PEO without diaziado compound, trimethylolpropyl triacrylate (TMPTA), and an acid functionalized monoacrylate (Photomer 4173) with photoinitiators. Both the primer coating 44 and the lubricious coating 34 are UV curable. Other hydrophilic coatings that form three dimensional networks under UV could also be used.

The following example illustrates a mixture for a primer coating 44 of the invention which provides for good adhesion, particularly on the filtering element 36. In addition to the specific formulation (with the amount of each component expressed as a weight percent of the solution mixture) used in the following example, Table B also gives example solution weight for the components which can be used in making the primer used in accordance with the present invention.

**TABLE B**

| | Specific wt% | General Weight % Range Formulations |
|---|---|---|
| IPA | 98.791% | about 95 to about 99 |
| Photomer 4173 | 1.185% | about 1 to about 3 |
| Benzophenone | 0.012% | about 0.01 to about 0.03 |
| Photomer 51 | 0.012% | about 0.01 to about 0.03 |

A solution mixture of formulation B listed in the above Table B was applied by spinning the solution mixture onto the filtering element 36 utilizing a high pressure nozzle and allowed to dry. The lubricious coating 34 was then applied to the primer coating 44. Additionally, the lubricious coating 34 can be provided with one or more therapeutic or diagnostic agents, and in one embodiment the agent elutes relatively quickly in a concentrated release from the lubricious coating upon hydration of the coating.

The following example illustrates a solution mixture for a lubricious coating 34 of the invention. In addition to the specific formulation (with the amount of each component expressed as a weight percent of the solution mixture) used in the following example, Table C also gives example solution weight percent ranges for the components which can be used in making coatings of the invention.

**TABLE C**

| | Specific Weight wt% | General Weight % Range Formulations |
|---|---|---|
| Ethanol | 91.886% | about 80 to about 95 |
| IPA | 2.964% | about 2 to about 5 |
| Water | 2.964% | about 2 to about 5 |
| PVP K90 | 1.778% | about 1 to about 4 |
| PHOTOMER® 4173 | 0.119% | about 0.05 to about 0.3 |
| TMPTA | 0.237% | about 0.1 to about 0,4 |
| PHOTOMER® 51 | 0,002% | about 0.001 to about 0.005 |
| Benzophenone | 0.002% | about 0.001 to about 0.005 |
| KBr | 0.030% | about 0.01 to about 0.06 |
| 4,4'-diazido-2,2-stilbenedisulfonic acid disodium salt hydrate | 0.018% | about 0.01 to about 0.04 |

A solution mixture of formulation C listed in the above Table C was then applied by spinning the solution mixture onto the filtering element 36 utilizing a high pressure nozzle to form the lubricious coating 34.

The guidewire and support frame of the embolic protection device 32 can be coated with the same primer coat polymer and lubricious coating as the filtering element 36. Alternatively, the guidewire and support frame could be coated with the primer and coating described above with respect to the guidewire 23 disclosed in FIGS. 5 and 6. Additionally, the primer coating and lubricious coating disclosed in TABLES B and C above could be alternatively applied to a catheter or other medical device.

## Claims

1. A medical device having a lubricious coating on at least a section of the device, the coating comprising:
a) a primer coating applied directly to at least a section of the device, the primer coating including an acid functionalized monoacrylate, a photoinitiator, isopropanol, benzophenone and a benzil dimethyl ketal, wherein the isopropanol makes up from 95 to 99 percent of the primer coating, the acid functionalized monoacrylate makes up from 1 to 3 percent of the primer coating, the benzophenone makes up from 0.01 to 0.2 percent of the primer coating and the benzil dimethyl ketal makes up from 0.01 to 0.2 percent of the primer coating ; and
b) a lubricious coating disposed on the primer coating, the lubricious coating including
i) polyvinylpyrrolidone, a diazido compound, trimethylolpropyl triacrylate, and an acid functionalized monoacrylate with photoinitiators, or
ii) polyethylene oxide, trimethylolpropyl triacrylate, and an acid functionalized monoacrylate with photoinitiators.

2. The medical device of claim 1, wherein the benzil dimethyl ketal is 2,2-dimethoxy-2 - phenyl acetophenone .

3. A method of providing a lubricious coating for a medical device, comprising: applying a primer coating directly to at least a section of the device, the primer coating including an acid functionalized monoacrylate, a photoinitiator, isopropanol, benzophenone and a benzil dimethyl ketal, wherein the isopropanol makes up from 95 to 99 percent of the primer coating, the acid functionalized monoacrylate makes up from 1 to 3 percent of the primer coating, the benzophenone makes up from 0.01 to 0.2 percent of the primer coating and the benzil dimethyl ketal makes up from 0.01 to 0.2 percent of the primer coating; and
applying a lubricious coating disposed on the primer coating, the lubricious coating including
i) polyvinylpyrrolidone, a diazido compound, trimethylolpropyl triacrylate, and an acid functionalized monoacrylate with photoinitiators, or
ii) polyethylene oxide, trimethylolpropyl triacrylate, and an acid functionalized monoacrylate with photoinitiators.

4. The method of claim 3 wherein the primer coating is applied to the medical device while the medical device is being spun.

5. The method of claim 4 wherein the lubricious coating is applied to the primer coating applied to at least a section of the medical device while the medical device is being spun.

6. The method of claim 3, wherein the benzil dimethyl ketal is 2,2-dimethoxy-2-phenyl acetophenone.

## Patentansprüche

1. Medizinische Vorrichtung mit einer gleitfähigen Beschichtung auf mindestens einem Abschnitt der Vorrichtung, wobei die Beschichtung umfasst:
a) eine Primärbeschichtung, die direkt auf mindestens einem Abschnitt der Vorrichtung aufgebracht ist, wobei die Primärbeschichtung ein säurefunktionalisiertes Monoacrylat, einen Photoinitiator, Isopropanol, Benzophenon und ein Benzil-Dimethylketal enthält, wobei das Isopropanol 95 bis 99 Prozent der Primärbeschichtung ausmacht, das säurefunktionalisierte Monoacrylat 1 bis 3 Prozent der Primärbeschichtung ausmacht, das Benzophenon 0,01 bis 0,2 Prozent der Primärbeschichtung ausmacht und das Benzil-Dimethyl-Ketal 0.01 bis 0,2 Prozent der Primärbeschichtung ausmacht und
b) eine Gleitschicht, die auf der Primärbeschichtung aufgebracht ist, wobei die Gleitschicht Folgendes umfasst
i) Polyvinylpyrrolidon, eine Diazidoverbindung, Trimethylolpropyltriacrylat und ein säurefunktionalisiertes Monoacrylat mit Photoinitiatoren oder
ii) Polyethylenoxid, Trimethylolpropyltriacrylat und ein säurefunktionalisiertes Monoacrylat mit Photoinitiatoren.

2. Medizinische Vorrichtung nach Anspruch 1, wobei das Benzil-Dimethylketal 2,2-dimethoxy-2-phenyl-acetophenon ist.

3. Verfahren zum Bereitstellen einer gleitfähigen Beschichtung für eine medizinische Vorrichtung, welches umfasst:
direktes Aufbringen einer Primärbeschichtung auf mindestens einem Abschnitt der Vorrichtung, wobei die Primärbeschichtung ein säurefunktionalisiertes Monoacrylat, einen Photoinitiator, Isopropanol, Benzophenon und ein Benzil-Dimethyl-Ketal enthält, wobei das Isopropanol 95 bis 99 Prozent der Primärbeschichtung ausmacht, das säurefunktionalisierte Monoacrylat 1 bis 3 Prozent der Primärbeschichtung ausmacht, das Benzophenon 0.01 bis 0,2 Prozent der Primärbeschichtung ausmacht und das Benzil-Dimethyl-Ketal 0.01 bis 0,2 Prozent der Primärbeschichtung ausmacht; und
Auftragen einer Gleitschicht auf die auf der Primärbeschichtung, wobei die Gleitschicht umfasst:
i) Polyvinylpyrrolidon, eine Diazidoverbindung, Trimethylolpropyltriacrylat und ein säurefunktionalisiertes Monoacrylat mit Photoinitiatoren oder
ii) Polyethylenoxid, Trimethylolpropyltriacrylat und ein säurefunktionalisiertes Monoacrylat mit Photoinitiatoren.

4. Verfahren nach Anspruch 3, bei dem die Primärbeschichtung auf die medizinische Vorrichtung aufgebracht wird, während die medizinische Vorrichtung rotiert wird.

5. Verfahren nach Anspruch 4, bei dem die gleitfähige Beschichtung auf die auf mindestens einen Abschnitt der medizinischen Vorrichtung aufgebrachte Primärbeschichtung aufgebracht wird, während die medizinische Vorrichtung rotiert wird.

6. Verfahren nach Anspruch 3, wobei das Benzil-Dimethylketal 2,2-Dimethoxy-2-phenylacetophenon ist.

## Revendications

1. Dispositif médical ayant un revêtement lubrifiant sur au moins une section du dispositif, le revêtement comprenant :
a) un revêtement d'apprêt appliqué directement sur au moins une section du dispositif, le revêtement d'apprêt comprenant un monoacrylate à fonctionnalité acide, un photoamorceur, de l'isopropanol, de la benzophénone et un benzyldiméthylcétal, dans lequel l'isopropanol représente de 95 à 99 % du revêtement d'apprêt, le monoacrylate à fonctionnalité acide représente de 1 à 3 % du revêtement d'apprêt, la benzophénone représente de 0,01 à 0,2 % du revêtement d'apprêt et le benzyldiméthylcétal représente de 0,01 à 0,2 % du revêtement d'apprêt ; et
b) un revêtement lubrifiant disposé sur le revêtement d'apprêt, le revêtement lubrifiant comprenant
i) de la polyvinylpyrrolidone, un composé diazido, du triacrylate de triméthylpropane, et un monoacrylate à fonctionnalité acide, avec des photoamorceurs, ou
ii) du poly(oxyde d'éthylène), du triacrylate de triméthylolpropane, et un monoacrylate à fonctionnalité acide, avec des photoamorceurs.

2. Dispositif médical selon la revendication 1, dans lequel le benzyldiméthylcétal est la 2,2-diméthoxy-2-phénylacétophénone.

3. Procédé de réalisation d'un revêtement lubrifiant pour un dispositif médical, comprenant :
l'application d'un revêtement d'apprêt directement sur au moins une section du dispositif, le revêtement d'apprêt comprenant un monoacrylate à fonctionnalité acide, un photoamorceur, de l'isopropanol, de la benzophénone et un benzyldiméthylcétal, dans lequel l'isopropanol représente de 95 à 99 % du revêtement d'apprêt, le monoacrylate à fonctionnalité acide représente de 1 à 3 % du revêtement d'apprêt, la benzophénone représente de 0,01 à 0,2 % du revêtement d'apprêt et le benzyldiméthylcétal représente de 0,01 à 0,2 % du revêtement d'apprêt ; et
l'application d'un revêtement lubrifiant disposé sur le revêtement d'apprêt, le revêtement lubrifiant comprenant
i) de la polyvinylpyrrolidone, un composé diazido, du triacrylate de triméthylpropane, et un monoacrylate à fonctionnalité acide, avec des photoamorceurs, ou
ii) du poly(oxyde d'éthylène), du triacrylate de triméthylolpropane, et un monoacrylate à fonctionnalité acide, avec des photoamorceurs.

4. Procédé selon la revendication 3, dans lequel le revêtement d'apprêt est appliqué sur le dispositif médical cependant que le dispositif médical est filé.

5. Procédé selon la revendication 4, dans lequel le revêtement lubrifiant est appliqué sur le revêtement d'apprêt appliqué à au moins une section du dispositif médical cependant que le dispositif médical est filé.

6. Procédé selon la revendication 3, dans lequel le benzyldiméthylcétal est la 2,2-diméthoxy-2-phénylacétophénone.
